# EUROPÄISCHE PATENTSCHRIFT

(11) **EP 0 732 893 B1**
(45) Veröffentlichungstag und Bekanntmachung des Hinweises auf die Patenterteilung: **29.04.1998**
(21) Anmeldenummer: 94930169.1
(22) Anmeldetag: 13.10.1994
(51) Int. Cl.: A61B 17/28

(54) **CHIRURGISCHES ROHRSCHAFTINSTRUMENT**
TUBULAR-HANDLED SURGICAL INSTRUMENT
INSTRUMENT CHIRURGICAL TUBULAIRE

(30) Priorität: 08.12.1993 DE 4341735
(43) Veröffentlichungstag der Anmeldung: 25.09.1996
(73) Patentinhaber: AESCULAP AG, 78532 Tuttlingen (DE)
(72) Erfinder: WEISSHAUPT, Dieter, D-78194 Immendingen (DE)
(74) Vertreter: Böhme, Ulrich
(86) Internationale Anmeldenummer: EP9403381
(87) Internationale Veröffentlichungsnummer: WO9515721

(56) Entgegenhaltungen:
- EP-A- 0 225 045
- EP-A- 0 541 930
- DE-U- 9 105 399

## Beschreibung

Die Erfindung betrifft ein chirurgisches Rohrschaftinstrument mit einem Rohr, an dessen Ende eine Halterung für ein Werkzeug lösbar gehalten ist, und mit einem in dem Rohr angeordneten und in diesem längsbewegbaren Betätigungselement zum Bewegen des Werkzeugs, wobei die Halterung eine in das Rohr einschiebbare Verlängerung trägt und wobei das Rohr an seinem die Verlängerung umgebenden Ende radial verschiebbare Teile aufweist, die im eingeschobenen Zustand durch Form- oder Kraftschluß die Verlängerung gegen axiale Verschiebung im Rohr sichern, im radial ausgeschobenen Zustand jedoch den Eingriff beenden und ein axiales Herausziehen der Verlängerung aus dem Rohr ermöglichen.

Chirurgische Rohrschaftinstrumente dieser Art werden für die verschiedensten Zwecke zum Schneiden, Festhalten, Koagulieren etc. benutzt. Normalerweise wird für jeden Einsatzzweck ein eigenes Instrument benötigt, es ist aber auch bekannt, die Werkzeuge an dem Rohr des Instruments lösbar anzuordnen. Zu diesem Zweck können beispielsweise die Werkzeughalterungen in das Rohr einschraubbar ausgebildet sein, wobei gleichzeitig im Endbereich des Rohrs eine Trennung des Betätigungselements erfolgt (Firmenprospekt der Firma Marlow Surgical Technologies Inc. "Nu-Tip").

Eine solche Verbindung der Werkzeughalterung mit dem Rohr ist relativ kompliziert, insbesondere ist es notwendig, auf Werkzeug und Rohr unter Umständen relativ große Kräfte beim Ein- und Ausschrauben auszuüben, außerdem benötigt das Gewinde eine gewisse Wandstärke des Rohrs, die bei der immer weiter fortschreitenden Miniaturisierung der Instrumente gegebenenfalls hinderlich sein kann.

Aus der EP 0 225 045 A1 ist ein chirurgisches Rohrschaftinstrument der eingangs beschriebenen Art bekannt, bei dem die Festlegung der Halterung des Werkzeugs allein durch radial ausfedernde Federzungen einer Hülse erfolgt, die Teil des Rohrschafts ist. Die Halterung wird durch kräftiges Herausziehen aus der Hülse von dieser gelöst, dabei federn die Federzungen nach außen. Es besteht allerdings die Gefahr, daß eine solche Lösung unbeabsichtigt hervorgerufen werden könnte. Um dies zu vermeiden, müssen die Federzungen sehr kräftig ausgebildet sein. Andererseits ist es dazu notwendig, große Kräfte auf die Halterung auszuüben, wenn sie vom Rohrschaft gelöst werden soll. Da es sich bei den beschriebenen Rohrschaftinstrumenten um sehr empfindliche und klein dimensionierte Instrumente handelt, ergibt sich dadurch die Gefahr einer Beschädigung.

Es ist Aufgabe der Erfindung, ein gattunsgemäßes Rohrschaftinstrument so auszubilden, daß die Lösung der Halterung aus dem Rohrschaft mit kleinen Kräften erfolgen kann, daß aber trotzdem die Halterung im Rohrschaft in axialer Richtung sicher gehalten wird und nicht unbeahsichtigt gelöst werden kann.

Diese Aufgabe wird bei einem Rohrschaftinstrument der eingangs beschriebenen Art erfindungsgemäß dadurch gelöst, daß auf dem Rohr längsverschieblich ein Außenrohr gelagert ist, welches über den Eingriffsbereich der Verlängerung in das Rohr geschoben, Rohr und Verlängerung in Form- oder Kraftschluß fixiert, aus diesem Bereich weggeschoben jedoch das radiale Ausschieben des Rohrs ermöglicht. Dadurch gelingt es, mit einer relativ materialsparenden und daher schwachen Ausbildung des Rohrs eine einwandfreie axiale Fixierung zu erhalten, da die radialen Kräfte zur Aufrechterhaltung des Form- oder Kraftschlusses durch das auf dem Rohr längsverschiebliche und dieses umgebende Außenrohr aufgenommen werden.

Besonders vorteilhaft ist eine Ausgestaltung, bei der das Rohr an seinem Ende nach innen abgebogene, durch Längsschlitze im Rohrmantel voneinander getrennte Zungen trägt, die im eingeschobenen Zustand mit ihrem nach innen gebogenen Ende in Rücksprünge auf dem Umfang des Verlängerung eingreifen. Bei dieser Lösung genügt es, das Rohr an seinem Ende durch Längsschlitze in Zungen zu unterteilen und diese an deren freiem Ende nach innen umzubiegen, um bereits auf diese Weise eine Verbindungsmöglichkeit zu erreichen. Beim Einschieben der Verlängerung werden die Zungen elastisch nach außen gebogen, und rasten anschließend mit ihrem nach innen umgebogenen Ende in die Rücksprünge der Verlängerung, so daß eine axiale Verschiebung verhindert wird.

Günstig ist es dabei, wenn die Rücksprünge durch eine Umfangsringnut in der Verlängerung gebildet werden.

Es ist weiterhin besonders vorteilhaft, wenn die Verlängerung kreiszylinderförmig ausgebildet ist, mit ihrem Außenmantel flächig an der Innenseite des Rohrs anliegt und im Abstand von ihrem freien Ende die Umfangsringnut trägt. Dadurch ergibt sich eine einwandfreie Lagerung der Halterung im Inneren des Rohrs, durch die Anlage des kreiszylinderförmigen Außenmantels der Verlängerung an der Innenseite des Rohrs über einen bestimmten Längenbereich erzielt man eine einwandfreie Führung der Verlängerung im Rohr und eine Verteilung der aufzunehmenden Kräfte über einen größeren Längenbereich.

Bei dem bevorzugten Ausführungsbeispiel kann dabei die Ringnut im Querschnitt sägezahnförmig ausgebildet sein, wobei die steile Flanke dem freien Ende der Verlängerung abgewandt ist. Beim Einschieben der Verlängerung gleiten dadurch die nach innen gebogenen freien Enden der elastischen Zungen des Rohrs in die Ringnut ein und stoßen an die steile Flanke an, dadurch wird die Einschubtiefe begrenzt. Beim Herausziehen gleiten die nach innen gebogenen Enden der Zungen an der flachen Flanke auf und ermöglichen so ein problemloses Herausziehen der Verlängerung aus dem Rohr.

Es kann weiterhin vorgesehen sein, daß die Verlängerung an ihrem freien Ende eine divergierende, in den kreiszylindrischen Bereich übergehende Aufgleitfläche trägt. Dies erleichtert das Einschieben der Verlängerung in das Rohr und das dadurch bedingte elastische Aufbiegen der zungenförmigen Rohrbereiche.

Vorteilhaft ist es dabei, wenn das Außenrohr über einen zylindrischen Bereich der Verlängerung schiebbar ist, der sich an den in das Rohr eintauchenden Eingriffsbereich der Verlängerung anschließt und der an der Innenwand des Außenrohrs anliegt. Dadurch ergibt sich zwischen Verlängerung und Außenrohr eine kraftschlüssige Verbindung, die zur Stabilisierung des gesamten Instruments beiträgt, da das Außenrohr auch flächig an dem die Verlängerung aufnehmenden Rohr anliegt. Das Außenrohr übernimmt somit eine zusätzliche Versteifungsfunktion zwischen Verlängerung einerseits und Rohr andererseits.

Bei einer besonders bevorzugten Ausführungsform ist vorgesehen, daß das Außenrohr aus einem elektrisch isolierenden Material besteht. Es wird dadurch möglich, die innerhalb des Außenrohres angeordneten Teile als elektrische Leiter zu verwenden, beispielsweise bei Instrumenten, die der Elektrokoagulation dienen. Trotzdem wird das umliegende Körpergewebe zuverlässig von den spannungsführenden Innenteilen des Instrumentes geschützt. Das Außenrohr übernimmt somit eine weitere Funktion, nämlich die der elektrischen Isolation des Rohres und der darinliegenden Teile gegenüber der Umgebung.

Dabei ist es vorteilhaft, wenn in das Außenrohr, das beispielsweise aus Kunststoffmaterial besteht, in dessen die Verlängerung überdeckenden Endabschnitt eine Verstärkungshülse eingesetzt ist. In diesem mechanisch besonders belasteten Bereich kann somit durch diese Verstärkungshülse die notwendige mechanische Festigkeit erreicht werden, obwohl das Kunststoffmaterial weiterhin die elektrischen Isolationseigenschaften beibehält.

Vorzugsweise besteht die Verstärkungshülse aus Metall.

Sie kann insbesondere an der Innenseite des Außenrohres angeordnet sein und stufenlos in den anschließenden Abschnitt des Außenrohres übergehen. Dadurch ergeben sich beim Aufschieben des Außenrohres auf das Rohr und die Verlängerung keinerlei Schwierigkeiten.

Es ist weiterhin vorteilhaft, wenn die Halterung außenseitig mit einem elektrisch isolierenden Material beschichtet ist, das sich bis an den vom Außenrohr überdeckten Bereich erstreckt. Damit ergibt sich auch im Bereich des Werkzeuges selbst eine elektrische Isolation gegenüber der Umgebung, die sich über die gesamte Länge des Instrumentes erstreckt.

Bei einer bevorzugten Ausführungsform sind das Rohr und die Verlängerung durch formschlüssigen Eingriff von Vor- und Rücksprüngen relativ zueinander gegen eine Verdrehung um die Rohrlängsachse gesichert. Insbesondere kann vorgesehen sein, daß die Verlängerung mindestens einen radial vorstehenden Vorsprung trägt, der in zum Ende des Rohres hin offene Längsschlitze im Rohr eingreift.

Damit ergibt sich die Möglichkeit, die Verbindung zwischen Werkzeughalterung einerseits und Rohr andererseits so auszubilden, daß nur auf die axiale Festlegung geachtet werden muß, nicht dagegen auf die Drehfestlegung. Diese wird durch die spezielle Drehsicherung übernommen. Dies vereinfacht die Ausbildung der Verbindung zwischen Werkzeughalterung einerseits und Rohr andererseits. Außerdem ergibt sich bei dieser Lösung gegenüber einer Verbindung durch ein Einschraubgewinde die Sicherheit gegen jede Verdrehung. Bei der Verwendung von Einschraubgewinden ist nicht auszuschließen, daß im Betrieb durch auftretende große Drehmomente die Schraubverbindung unbeabsichtigt gelöst wird. Diese Gefahr entfällt bei der beschriebenen Neukonstruktion.

Die nachfolgende Beschreibung bevorzugter Ausführungsformen der Erfindung dient im Zusammenhang mit der Zeichnung der näheren Erläuterung. Es zeigen:

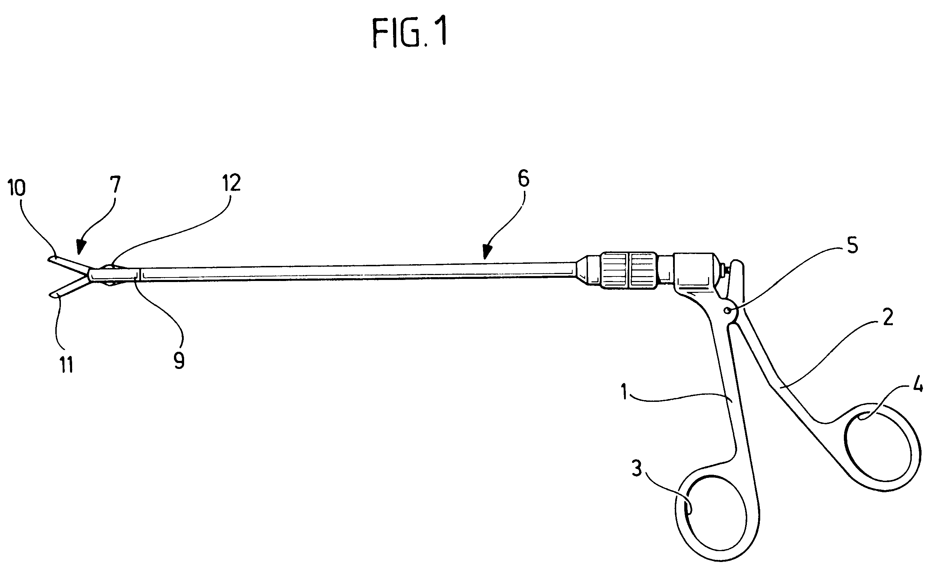

- Figur 1:: eine schematische Seitenansicht eines Rohrschaftinstrumentes;
- Figur 2:: eine teilweise aufgebrochene Seitenansicht des vorderen Teils bei der bevorzugten Ausführungsform eines Rohrschaftinstrumentes mit am Rohr festgelegtem Werkzeug;
- Figur 3:: eine Ansicht ähnlich Figur 2 mit vom Rohr gelöstem Werkzeug und
- Figur 4:: eine vergrößerte Ansicht des vorderen Teils einer weiteren bevorzugten Ausführungsform eines Rohrschaftinstrumentes mit elektrischer Isolierung (ohne Werkzeug).

Das chirurgische Rohrschaftinstrument wird im folgenden beschrieben am Beispiel eines Instrumentes, das mit zwei gegeneinander verschwenkbaren Griffbranchen betätigbar ist. Es versteht sich jedoch, daß jede Art von Instrument durch die Erfindung betroffen ist, bei dem in einem Rohr ein Betätigungselement verschiebbar gelagert ist, welches ein Werkzeug am Ende des Rohres betätigt. Der Antrieb des Betätigungselementes kann auch in anderer Weise als durch gegeneinander verschwenkbare Griffbranchen erfolgen, beispielsweise durch unmittelbare Verschiebung des Betätigungselementes oder durch spezielle motorische Antriebsmittel.

Das in der Zeichnung beschriebene Rohrschaftinstrument umfaßt zwei gegeneinander verschwenkbar gelagerte Griffbranchen 1, 2 mit je einer Fingeröffnung 3 bzw. 4, die an einer Lagerstelle 5 schwenkbar miteinander verbunden sind.

Eine der Griffbranchen 1 trägt ein längliches Rohr 6, an dessen freiem Ende ein Werkzeug 7 gehalten ist, das beispielsweise als Zange oder Schere ausgebildet sein kann.

Im Inneren des Rohres 6 ist ein Betätigungselement in Form einer Schub- und Zugstange 8 angeordnet, die über Gelenkverbindungen einerseits mit dem Werkzeug 7 und andererseits mit der anderen Griffbranche 2 verbunden ist. Durch Öffnen und Schließen der Griffbranchen 1 und 2 wird die Schub- und Zugstange 8 in dem Rohr 6 längsverschoben und betätigt dadurch das Werkzeug 7.

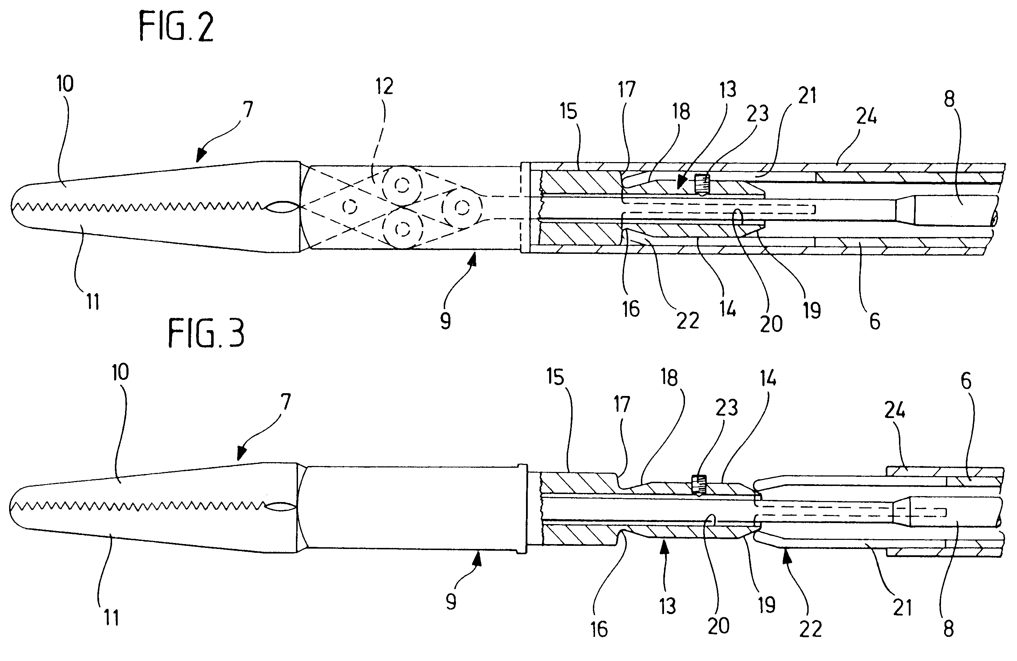

Anhand der Figuren 2 bis 4 wird der Aufbau des Rohrschaftinstrumentes im Bereich der Übergangsstelle zwischen dem Rohr 6 einerseits und dem Werkzeug 7 andererseits genauer erläutert.

Das Werkzeug 7 umfaßt eine Halterung 9, an der bewegliche Teile 10, 11 des Werkzeuges drehbar gelagert sind. Außerdem nimmt die Halterung 9 Getriebemittel 12 auf, die drehbar mit der Schub- und Zugstange 8 verbunden sind und die eine Bewegung der Schub- und Zugstange in eine Drehbewegung der Teile 10 und 11 übersetzen.

Die Halterung 9 geht in ihrer dem Rohr 6 zugewandten Seite in eine stufige, kreiszylindrische Verlängerung 13 über, die einen dem Ende der Verlängerung 13 benachbarten Abschnitt 14 mit geringerem Außendurchmesser und einen daran anschließenden Abschnitt 15 mit größerem Außendurchmesser aufweist. Im Übergangsbereich zwischen den beiden Abschnitten 14 und 15 befindet sich in der Verlängerung 13 eine Umfangsnut 16, die durch einen konischen Verlauf der Mantelfläche des Abschnittes 14 ausgebildet wird. Dadurch erhält die Umfangsnut 16 einen Sägezahnquerschnitt mit einer durch die Stufe zwischen den Abschnitten 14 und 15 gebildeten steilen Flanke 17 und mit einer in die Zylinderfläche des Abschnittes 14 übergehenden flachen Flanke 18.

Am freien Ende der Verlängerung 13 ist eine konische Aufgleitfläche 19 ausgebildet, die ebenfalls in die Zylinderfläche des Abschnittes 14 übergeht. Die Verlängerung 13 nimmt eine zentrale Bohrung 20 auf, in der die Schub- und Zugstange 8 bis zu den Getriebeteilen 12 verlaufend angeordnet ist. Diese Schub- und Zugstange 8 kann ein durchgehender Stab sein, im dargestellten Ausführungsbeispiel ist sie zweistückig ausgebildet und erweitert sich im Inneren des Rohres 6, dies ist nicht unbedingt notwendig.

Das Rohr 6 ist an seinem freien Ende durch mehrere Längsschlitze 21 in zungenförmige Wandbereiche 22 unterteilt, die am freien Ende des Rohres 6 geringfügig radial nach innen abgebogen sind. Die Abwinkelung dieser zungenförmigen Wandbereiche 22 entspricht dabei im wesentlichen dem Winkel der flachen Flanke 18 der Umfangsnut 16, außerdem ist die Länge des abgewinkelten Bereiches so gewählt, daß der abgebogene Teil des zungenförmigen Wandbereiches 22 etwa gleichlang ist wie die flache Flanke 18 der Umfangsnut 16. Der Außendurchmesser des Abschnittes 14 entspricht dem Innendurchmesser des Rohres 6. In den Abschnitt 14 ist in radialer Richtung eine Schraube 23 eingedreht, die geringfügig über den Außendurchmesser des Abschnittes 14 vorsteht und deren Durchmesser der Breite der Längsschlitze 21 im Rohr 6 entspricht.

Auf dem Rohr 6 ist ein Außenrohr 24 längsverschieblich angeordnet, dessen Innendurchmesser dem Außendurchmesser des Rohres entspricht. Dieser Innendurchmesser entspricht außerdem dem Außendurchmesser des Abschnittes 15 der Verlängerung.

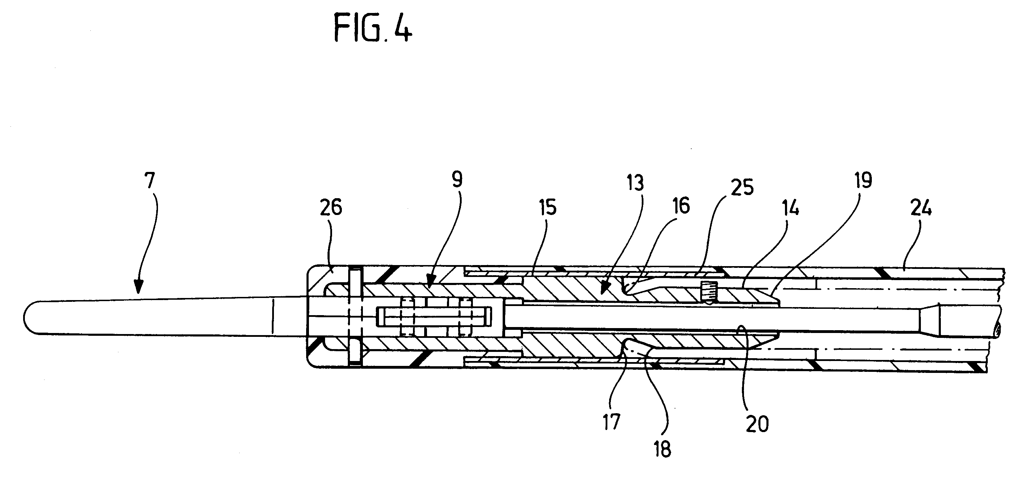

Dieses Außenrohr kann entsprechend der Darstellung in den Figuren 2 und 3 aus Metall bestehen, es ist jedoch günstig, wenn dieses Außenrohr 24 entsprechend der Ausführungsform der Figur 4 aus einem elektrisch isolierenden Material besteht, beispielsweise aus Kunststoff. In diesem Falle wird in den endnahen Bereich des Außenrohres 24 eine Verstärkungshülse 25 eingesetzt (Figur 4), die beispielsweise aus Metall bestehen kann. Vorzugsweise wird die Verstärkungshülse 25 an der Innenseite des Außenrohres 24 angeordnet und so eingepaßt, daß die Innenwand des Außenrohres 24 stufenlos ist. Bei einem Ausführungsbeispiel mit einem Außenrohr aus elektrisch isolierendem Material ist es weiterhin vorteilhaft, wenn die Halterung 9 außenseitig mit einem elektrisch isolierenden Material 26 beschichtet ist, beispielsweise ebenfalls mit Kunststoff. Dieses erstreckt sich vorzugsweise bis in den Abschnitt 15 der Verlängerung 13, so daß das elektrisch isolierende Außenrohr 24 unmittelbar an dieses elektrisch isolierende Material 26 anschließen kann, teilweise sogar unter Überdeckung.

Bei der Verbindung eines Werkzeuges 7 mit einem Rohr 6 wird zunächst das auf dem Rohr 6 verschiebbar gelagerte Außenrohr 24 gegenüber dem Rohr 6 soweit zurückgeschoben, daß die zungenförmigen Wandbereiche 22 des Rohres 6 freigegeben werden und daher elastisch nach außen verbogen werden können. In der in Figur 3 dargestellten Weise wird ein Werkzeug 7 so an das Rohr 6 herangeführt, daß die Aufgleitfläche 19 der Verlängerung 13 an den freien Enden der zungenförmigen Wandbereiche 22 anliegt und diese dadurch elastisch radial nach außen verschiebt. Die zungenförmigen Wandbereiche 22 gleiten beim weiteren Einschieben des Abschnittes 14 in das Rohr 6 an dessen Außenfläche entlang, bis die abgebogenen Teile der zungenförmigen Wandbereiche 22 in die Umfangsnut 16 gelangen. Die Verschiebebewegung wird durch Anlage der zungenförmigen Wandbereiche 22 an der steilen Flanke 17 begrenzt. Wenn diese Lage erreicht ist, liegen die zungenförmigen Wandbereiche 22 mit ihrem zylindrischen Teil an der zylindrischen Mantelfläche des Abschnittes 14 flächig an, während die abgebogenen Teile der zungenförmigen Wandbereiche 22 in die Umfangsnut 16 eingreifen, vorzugsweise unter flächiger Anlage an der flachen Flanke 18, wie dies in Figur 2 dargestellt ist. Die Verlängerung wird dabei durch den Eingriff der Schraube 23 in einen der Längsschlitze 21 gegen eine Verdrehung gegenüber dem Rohr 6 gesichert. Zur dauerhaften Festlegung dieser Verbindung wird anschließend das Außenrohr 24 soweit über das Rohr 6 geschoben, daß die zungenförmigen Wandbereiche 22 in radialer Richtung festgelegt werden, sich also nicht mehr radial nach außen bewegen können. Dabei wird das Außenrohr 24 vollständig über den Abschnitt 15 der Verlängerung 13 geschoben, so daß dadurch eine Aussteifung im Verbindungsbereich erfolgt. Halterung 9 und Rohr 6 sind dadurch in axialer Richtung unverschieblich festgelegt, weiterhin ergibt sich durch das koaxiale Ineinandergreifen des Rohres 6, des Außenrohres 24 und der Verlängerung 13 auch eine biegesteife Verbindung zwischen Halterung 9 und dem Rohr 6.

Diese Verbindung kann in einfachster Weise wieder gelöst werden, wenn das Außenrohr 24 zurückgezogen wird und die zungenförmigen Wandbereiche 22 wieder freigibt. Es ist dann möglich, das Werkzeug 7 aus dem Rohr 6 herauszuziehen, wobei die Schub- und Zugstange 8 mit herausgezogen wird.

Bei dem Ausführungsbeispiel der Figur 4 ergibt sich im montierten Zustand bei vorgeschobenem Außenrohr 24 eine vollständige elektrisch isolierende Ummantelung des gesamten Schaftteiles des Instrumentes, so daß dieses Instrument für elektrische Bearbeitungsvorgänge eingesetzt werden kann.

## Patentansprüche

1. Chirurgisches Rohrschaftinstrument mit einem Rohr, an dessen Ende eine Halterung für ein Werkzeug lösbar gehalten ist, und mit einem in dem Rohr angeordneten und in diesem längsbewegbaren Betätigungselement zum Bewegen des Werkzeugs, wobei die Halterung (9) eine in das Rohr (6) einschiebbare Verlängerung (13) trägt und wobei das Rohr (6) an seinem die Verlängerung (13) umgebenden Ende radial verschiebbaren Teile (22) aufweist, die im eingeschobenen Zustand durch Form- oder Kraftschluß die Verlängerung (13) gegen axiale Verschiebung im Rohr (6) sichern, im radial ausgeschobenen Zustand jedoch den Eingriff beenden und ein axiales Herausziehen der Verlängerung (13) aus dem Rohr (6) ermöglichen, dadurch gekennzeichnet, daß auf dem Rohr (6) längsverschieblich ein Außenrohr (24) gelagert ist, welches über einen Eingriffsbereich der Verlängerung (13) in das Rohr (6) geschoben Rohr (6) und Verlängerung (13) in Form- oder Kraftschluß fixiert, aus diesem Bereich weggeschoben jedoch das radiale Ausschieben der radial verschiebbaren Teile (22) ermöglicht.

2. Instrument nach Anspruch 1, dadurch gekennzeichnet, daß das Rohr (6) an seinem Ende nach innen abgebogene, durch Längsschlitze (21) im Rohrmantel voneinander getrennte Zungen (22) trägt, die im eingeschobenen Zustand mit ihrem nach innen gebogenen Ende in Rücksprünge (16) auf dem Umfang der Verlängerung (13) eingreifen.

3. Instrument nach Anspruch 2, dadurch gekennzeichnet, daß die Rücksprünge (16) durch eine Umfangsringnut in der Verlängerung (13) gebildet werden.

4. Instrument nach den Ansprüchen 2 und 3, dadurch gekennzeichnet, daß die Verlängerung (13) kreiszylinderförmig ausgebildet ist, mit ihrem Außenmantel flächig an der Innenseite des Rohrs (6) anliegt und im Abstand von ihrem freien Ende die Umfangsringnut trägt.

5. Instrument nach Anspruch 4, dadurch gekennzeichnet, daß die Umfangsringnut im Querschnitt sägezahnförmig ausgebildet ist, wobei die steile Flanke (17) dem freien Ende der Verlängerung (13) abgewandt ist.

6. Instrument nach Anspruch 4 oder 5, dadurch gekennzeichnet, daß die Verlängerung (13) an ihrem freien Ende eine divergierende, in den kreiszylindrischen Bereich (14) übergehende Aufgleitfläche (19) trägt.

7. Instrument nach einem der voranstehenden Ansprüche, dadurch gekennzeichnet, daß das Außenrohr (24) über einen zylindrischen Bereich (15) der Verlängerung (13) schiebbar ist, der sich an den in das Rohr (6) eintauchenden Eingriffsbereich (14) der Verlängerung (13) anschließt und der an der Innenwand des Außenrohrs (24) anliegt.

8. Instrument nach einem der voranstehenden Ansprüche, dadurch gekennzeichnet, daß das Außenrohr (24) aus einem elektrisch isolierenden Material besteht.

9. Instrument nach Anspruch 8, dadurch gekennzeichnet, daß in das Außenrohr (24) in dessen die Verlängerung (13) überdeckenden Endabschnitt eine Verstärkungshülse (25) eingesetzt ist.

10. Instrument nach Anspruch 9, dadurch gekennzeichnet, daß die Verstärkungshülse (25) aus Metall besteht.

11. Instrument nach Anspruch 9 oder 10, dadurch gekennzeichnet, daß die Verstärkungshülse (25) an der Innenseite des Außenrohrs (24) angeordnet ist und stufenlos in den anschließenden Abschnitt des Außenrohrs (24) übergeht.

12. Instrument nach einem der Ansprüche 8 bis 11, dadurch gekennzeichnet, daß die Halterung (9) außenseitig mit einem elektrisch isolierenden Material (26) beschichtet ist, das sich bis in den vom Außenrohr (24) überdeckten Bereich erstreckt.

13. Instrument nach einem der voranstehenden Ansprüche, dadurch gekennzeichnet, daß Rohr (6) und Verlängerung (13) durch formschlüssigen Eingriff von Vor- und Rücksprüngen (23, 21) relativ zueinander gegen eine Verdrehung um die Rohrlängsachse gesichert sind.

14. Instrument nach Anspruch 13, dadurch gekennzeichnet, daß die Verlängerung (13) mindestens einen radial vorstehenden Vorsprung (23) trägt, der in zum Ende des Rohrs (6) hin offene Längsschlitze (21) im Rohr (6) eingreift.

## Claims

1. A tubular-shaft surgical instrument, comprising a tube, at the end of which a mounting for an implement is detachably held, and an actuating member arranged in the tube so as to be longitudinally displaceable therein for moving the implement, wherein the mounting (9) carries an extension (13) insertable into the tube (6) and wherein the end of the tube (6) surrounding the extension (13) has radially displaceable parts (22) which, when pushed in, secure the extension (13) against axial displacement in the tube (6) by means of a positive or frictional connection, but, when pushed radially outwards, release the engagement and allow the extension (13) to be withdrawn axially from the tube (6), characterised in that an outer tube (24) is longitudinally displaceably mounted on the tube (6) and, when pushed over an engagement region of the extension (13) in the tube (6), fixes the tube (6) and the extension (13) by means of a positive or frictional connection, but, when pushed out of this region, allows the radially displaceable parts (22) to be pushed radially outwards.

2. An instrument according to claim 1, characterised in that the end of the tube (6) carries inwardly bent tongues (22) separated from one another by longitudinal slots (21) in the tubular casing, the inwardly bent end of the tongues (22), when pushed in, engaging in recesses (16) on the circumference of the extension (13).

3. An instrument according to claim 2, characterised in that the recesses (16) are formed by a circumferential annular groove in the extension (13).

4. An instrument according to claims 2 and 3, characterised in that the extension (13) is formed in the manner of a circular cylinder, rests with its outer surface flat against the inside of the tube (6) and carries the circumferential annular groove at a distance from its free end.

5. An instrument according to claim 4, characterised in that the circumferential annular groove is saw-tooth-shaped in cross-section, the steep flank (17) being remote from the free end of the extension (13).

6. An instrument according to claim 4 or 5, characterised in that the free end of the extension (13) carries a diverging ascending surface (19) merging into the circular cylindrical region (14).

7. An instrument according to any one of the preceding claims, characterised in that the outer tube (24) is slidable over a cylindrical region (15) of the extension (13), the cylindrical region (15) adjoining the engagement region (14) of the extension (13) extending into the tube (6), and resting against the inner wall of the outer tube (24).

8. An instrument according to any one of the preceding claims, characterised in that the outer tube (24) comprises an electrically insulating material.

9. An instrument according to claim 8, characterised in that a reinforcing sleeve (25) is inserted into the outer tube (24) in the end portion thereof covering the extension (13).

10. An instrument according to claim 9, characterised in that the reinforcing sleeve (25) is made of metal.

11. An instrument according to claim 9 or 10, characterised in that the reinforcing sleeve (25) is arranged on the inside of the outer tube (24) and merges continuously into the adjoining portion of the outer tube (24).

12. An instrument according to any one of claims 8 to 11, characterised in that the outside of the mounting (9) is covered with an electrically insulating material (26) extending into the region covered by the outer tube (24).

13. An instrument according to any one of the preceding claims, characterised in that the tube (6) and the extension (13) are mutually secured against rotation about the longitudinal axis of the tube by the positive engagement of projections and recesses (23, 21).

14. An instrument according to claim 13, characterised in that the extension (13) carries at least one radially extending projection (23) engaging in longitudinal slots (21) in the tube (6), the longitudinal slots (21) being open to the end of the tube (6).

## Revendications

1. Instrument chirurgical à fût tubulaire comportant un tube à l'extrémité duquel est retenue de façon détachable une monture pour un outil, et comportant un élément d'actionnement destiné à déplacer l'outil, qui est agencé dans le tube et qui est mobile longitudinalement dans celui-ci, dans lequel la monture (9) porte un prolongement (13) enfichable dans le tube (6) et le tube (6) présente à son extrémité entourant le prolongement (13) des pièces radialement mobiles (22) qui bloquent à l'état enfiché le prolongement (13) à l'encontre d'un déplacement axial dans le tube (6) par coopération de formes ou de forces, mais qui terminent l'engagement à l'état radialement déployé et permettent une extraction axiale du prolongement (13) hors du tube (6), caractérisé en ce qu'un tube extérieur (24) est monté en déplacement longitudinal sur le tube (6) et est enfilé sur une région d'engagement du prolongement (13) dans le tube (6) et fixe le tube (6) et le prolongement (13) par coopération de formes ou de forces, mais qui, lorsqu'il est éloigné de cette région, permet l'extraction radiale des pièces mobiles (22).

2. Instrument selon la revendication 1, caractérisé en ce que le tube (6) porte à son extrémité des languettes (22) coudées vers l'intérieur et séparées les unes des autres par des fentes longitudinales (21) dans l'enveloppe de tube, qui, à l'état enfilé, s'engagent par leur extrémité coudée vers l'intérieur dans des retraits (16) sur la périphérie du prolongement (13).

3. Instrument selon la revendication 2, caractérisé en ce que les retraits (16) sont formés par une gorge annulaire périphérique dans le prolongement (13).

4. Instrument selon les revendications 2 et 3, caractérisé en ce que le prolongement (13) est réalisé sous forme cylindrique circulaire, en ce qu'il s'appuie par son enveloppe extérieure à plat contre la face intérieure du tube (6), et en ce qu'il porte la gorge annulaire périphérique à distance de son extrémité libre.

5. Instrument selon la revendication 4, caractérisé en ce que la gorge annulaire périphérique est réalisée avec une section transversale en forme de dent de scie, le flanc raide (17) étant détourné de l'extrémité libre du prolongement (13).

6. Instrument selon l'une ou l'autre des revendications 4 et 5, caractérisé en ce que le prolongement (13) porte à son extrémité libre une surface de coulissement (19) divergente qui se transforme en la région cylindrique circulaire (14).

7. Instrument selon l'une quelconque des revendications précédentes, caractérisé en ce que le tube extérieur (24) est susceptible d'être enfilé sur une région cylindrique (15) du prolongement (13) plongeant dans le tube (6), qui se raccorde à la région d'engagement (14) du prolongement (13) et qui s'appuie contre la paroi intérieure du tube extérieur (24).

8. Instrument selon l'une quelconque des revendications précédentes, caractérisé en ce que le tube extérieur (24) est constitué en un matériau électriquement isolant.

9. Instrument selon la revendication 8, caractérisé en ce qu'une douille de renforcement (25) est mise en place dans le tube extérieur (24) dans son tronçon d'extrémité recouvrant le prolongement (13).

10. Instrument selon la revendication 9, caractérisé en ce que la douille de renforcement (25) est constituée en métal.

11. Instrument selon l'une ou l'autre des revendications 9 et 10, caractérisé en ce que la douille de renforcement (25) est agencée sur la face intérieure du tube extérieur (24) et se transforme sans gradin en le tronçon qui s'y raccorde du tube extérieur (24).

12. Instrument selon l'une quelconque des revendications 8 à 11, caractérisé en ce que la monture (9) est revêtue sur la face extérieure d'un matériau électriquement isolant (26) qui s'étend jusque dans la région recouverte par le tube extérieur (24).

13. Instrument selon l'une quelconque des revendications précédentes, caractérisé en ce que le tube (6) et le prolongement (13) sont bloqués l'un par rapport à l'autre à l'encontre d'une rotation autour de l'axe longitudinal de tube par engagement en coopération de formes des saillies et des retraits (23, 21).

14. Instrument selon la revendication 13, caractérisé en ce que le prolongement (13) porte au moins une saillie (23) dépassant radialement, qui s'engage dans des fentes longitudinales (21) dans le tube (6) ouvertes vers l'extrémité du tube (6).
